# EUROPEAN PATENT APPLICATION

(11) **EP 4 220 644 A1**
(43) Date of publication of application: **02.08.2023**
(21) Application number: 22198688.8
(22) Date of filing: 29.09.2022
(51) Int. Cl.: G16B 30/00, G16B 40/20

(54) **FEATURE AMOUNT CALCULATION PROGRAM, FEATURE AMOUNT CALCULATION METHOD, AND FEATURE AMOUNT CALCULATION DEVICE**

(30) Priority: 26.01.2022 JP 2022010118
(71) Applicant: FUJITSU LIMITED, Kawasaki-shi, Kanagawa 211-8588 (JP)
(72) Inventor: Terashima, Chieko, Kawasaki-shi, Kanagawa, 211-8588 (JP)
(74) Representative: Hoffmann Eitle

(57) **Abstract**

A feature amount calculation program for causing a computer to execute processing including: receiving structure specifying information indicating a type of each of atomic groups and a sequence of the atomic groups regarding a cyclic molecule in which the atomic groups classified into a plurality of types is cyclically sequenced; specifying an optional first type and an optional second type in the plurality of types; specifying, based on the structure specifying information, one or more of first atomic groups classified into the first type and one or more of second atomic groups classified into the second type out of the atomic groups; and calculating, based on the structure specifying information, a number of pairs of the first atomic group and the second atomic group in which a mutual distance in the sequence between the first atomic group and the second atomic group is a distance.

## Description

### FIELD

The embodiments relate to a feature amount calculation program, a feature amount calculation method, and a feature amount calculation device.

### BACKGROUND

Recently, in a field of drug discovery, machine learning has been attracting attention as a method for searching for candidate molecules, and a technology of specifying a feature amount that may be handled by machine learning on the basis of a molecular structure is being considered.

As an example, for example, a method for using a fingerprint as a feature amount, a method for representing a feature amount by considering a molecular sequence as a structure having a beginning and an end and the like are known.

Japanese National Publication of International Patent Application No. 2012-509848 and Japanese National Publication of International Patent Application No. 2020-517290 are disclosed as related art.

Tajimi et al. BMC Bioinformatics 2018, 19 (Suppl 19): 527, X. Yang et al. / Computational and Structural Biotechnology Journal 18 (2020) 153-161, and Carhart et al., J. Chem. Inf., 1985 are also disclosed as related art.

### SUMMARY

### TECHNICAL PROBLEM

The conventional technology described above was insufficient to reflect a molecular structure to a feature amount with high accuracy in a case where a partial structure of the molecule has a specific sequence, in a case where the molecule includes a cyclic structure and the like.

In one aspect, an object of the present embodiments is to reflect the molecular structure to the feature amount with high accuracy.

### SOLUTION TO PROBLEM

According to an aspect of the embodiments, there is provided a feature amount calculation program comprising instructions which, when executed by a computer, cause the computer to execute processing including: receiving structure specifying information that specifies a type of each of a plurality of atomic groups and a sequence of the plurality of atomic groups regarding a cyclic molecule in which the plurality of atomic groups classified into a plurality of types is cyclically sequenced; specifying an optional first type and an optional second type in the plurality of types; specifying, on the basis of the structure specifying information, one or a plurality of first atomic groups classified into the first type and one or a plurality of second atomic groups classified into the second type out of the plurality of atomic groups; and calculating, on the basis of the structure specifying information, a number of pairs of the first atomic group and the second atomic group in which a mutual distance in the sequence between the first atomic group and the second atomic group is a predetermined distance.

### ADVANTAGEOUS EFFECTS OF INVENTION

A feature of a molecular structure is reflected to a feature amount with high accuracy.

### BRIEF DESCRIPTION OF DRAWINGS

FIG. 1 is a diagram illustrating an example of a feature amount calculation device;
FIG. 2(i.e., FIGs. 2A and 2B) is a diagram for illustrating a feature amount of a first embodiment;
FIG. 3 is a diagram illustrating an example of a hardware configuration of the feature amount calculation device;
FIG. 4 is a diagram for illustrating a function of a feature amount calculation unit of the first embodiment;
FIG. 5(i.e., FIGs. 5A and 5B) is a diagram for illustrating structure specifying information;
FIG. 6 is a flowchart for illustrating processing of the feature amount calculation device of the first embodiment;
FIG. 7A is a first diagram for illustrating processing using the feature amount;
FIG. 7B is a second diagram for illustrating processing using the feature amount;
FIG. 7C is a third diagram for illustrating processing using the feature amount;
FIGs. 8A to 8C are diagrams for illustrating a feature amount of a second embodiment;
FIG. 9 is a diagram for illustrating a function of a feature amount calculation unit of the second embodiment; and
FIG. 10 is a flowchart for illustrating processing of a feature amount calculation device of the second embodiment.

### DESCRIPTION OF EMBODIMENTS

### (First Embodiment)

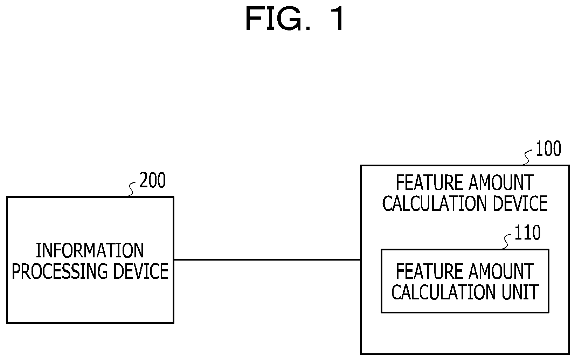

Hereinafter, embodiments are described with reference to the drawings. FIG. 1 is a diagram illustrating an example of a feature amount calculation device.

A feature amount calculation program is installed in a feature amount calculation device 100 of this embodiment, and a function of a feature amount calculation unit 110 is implemented by executing the feature amount calculation program. The feature amount calculation unit 110 is described later in detail.

The feature amount calculation device 100 of this embodiment is connected to, for example, an information processing device 200 and the like via a network and the like.

When structure specifying information specifying a molecular structure is input from the information processing device 200, the feature amount calculation device 100 of this embodiment calculates a feature amount indicating the molecular structure using the structure specifying information by the feature amount calculation unit 110 and outputs the same to the information processing device 200.

Structure specifying information 10 is information specifying a structure of a molecule in which a plurality of atomic groups is cyclically sequenced. The structure specifying information 10 is described later in detail.

When the structure specifying information 10 is input, the feature amount calculation device 100 acquires information indicating the number of atomic groups of a specific type arranged n atomic groups away for each of a plurality of atomic groups included in the structure specifying information 10 and output the same to the information processing device 200.

The information processing device 200 may include a learning unit and may perform machine learning using the feature amount output from the feature amount calculation device 100. For example, a feature amount 30 of this embodiment may be used to predict a substance amount required for drug discovery and the like.

In this embodiment, by expressing the feature amount in this manner, it is possible to reflect the fact that the specific atomic group is included in the molecule and the fact that the plurality of atomic groups is cyclically sequenced to the feature amount of the molecule. Therefore, according to this embodiment, the molecular structure may be reflected to the feature amount with high accuracy.

Note that, in this embodiment, the atomic group indicates a partial structure in the molecule. For example, the partial structure (atomic group) of this embodiment is an amino acid. Furthermore, the molecule in this embodiment indicates a cyclic peptide. For example, the cyclic peptide is a molecule in which a plurality of amino acids is cyclically sequenced.

Types of amino acids include, for example, aspartic acid, leucine, lysine and the like. In the following description, aspartic acid might be expressed as "asp", leucine as "leu", and lysine as "lys".

Note that, in the example in FIG. 1, the structure specifying information 10 is assumed to be input from the information processing device 200, but this is not limited thereto. The structure specifying information 10 may be directly input to the feature amount calculation device 100.

Furthermore, in the example in FIG. 1, the feature amount 30 is assumed to be output to the information processing device 200, but this is not limited thereto. The feature amount 30 may be output to a device other than the information processing device 200. Furthermore, an output destination of the feature amount 30 may be, for example, a learning device that performs machine learning using the feature amount 30.

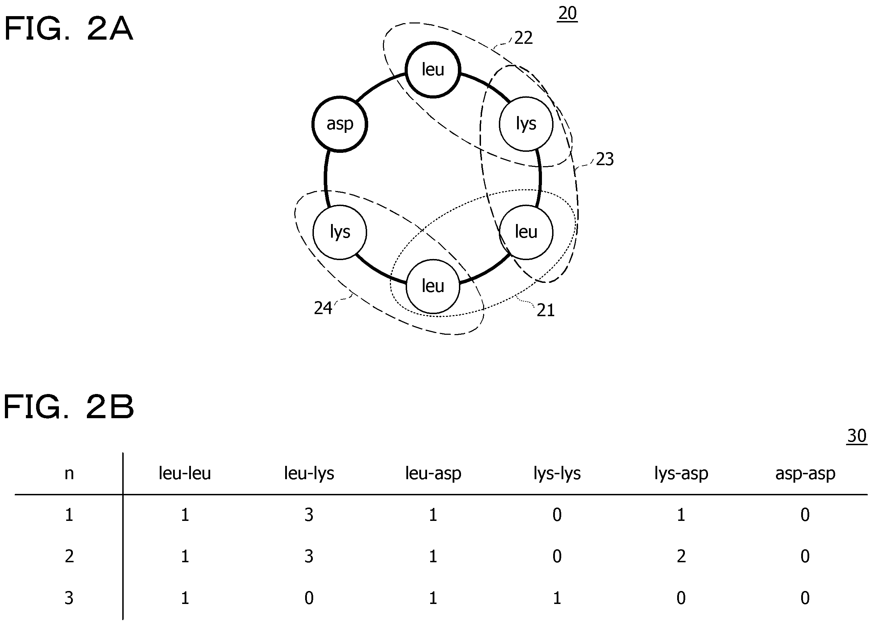

Hereinafter, the feature amount of this embodiment is described with reference to FIG. 2. FIG. 2 (i.e., FIGs. 2A and 2B) is a diagram for illustrating the feature amount of a first embodiment. FIG. 2A illustrates an example of the cyclic peptide, and FIG. 2B illustrates an example of the feature amount 30.

In this embodiment, for each amino acid being each atomic group included in a cyclic peptide 20, the number of pairs of a certain amino acid and an amino acid located in an n-th position from the amino acid in a sequence of the cyclic peptide 20 is counted. Then, in this embodiment, a matrix in which a value of n is made a row, information indicating types of amino acids included in the pair is made a column, and the number of pairs is made a component is made the feature amount.

Here, in this specification, the value of n is referred to as a "distance" between the amino acids in the cyclic peptide 20. In this case, the feature amount 30 of this embodiment may be said to be information including, for each amino acid in the cyclic peptide 20, the value of n indicating a distance between a certain amino acid and another amino acid, and the number of other amino acids arranged at a distance n from a certain amino acid. For example, this information may be said to be information indicating a positional relationship between each amino acid and another amino acid in a sequence of amino acids included in the cyclic peptide 20.

Furthermore, the feature amount 30 includes information indicating a type of a certain amino acid and a type of another amino acid located in an n-th position from the certain amino acid.

For example, the feature amount 30 of this embodiment may be said to be the information indicating a positional relationship between each amino acid and another amino acid in the sequence of amino acids included in the cyclic peptide 20 and the information indicating a type of each amino acid and a type of another amino acid.

Note that, the another amino acid in this embodiment may be the same type of amino acid as the certain amino acid, or may be a different type of amino acid.

The cyclic peptide 20 illustrated in FIG. 2A has a structure in which leucine (leu), aspartic acid (asp), and lysine (lys) are cyclically sequenced.

Therefore, in this embodiment, each of the number of pairs (leu-leu) of leucine and leucine located in an n-th position from leucine, the number of pairs (leu-asp) of leucine and aspartic acid located in an n-th position from leucine, and the number of pairs (leu-lys) of leucine and lysine located in an n-th position from leucine is counted.

For example, in this embodiment, the number of other amino acids arranged in the position at the distance n from leucine is counted. Here, the other amino acids include leucine, aspartic acid, and lysine.

Moreover, in this embodiment, the number of pairs (asp-asp) of aspartic acid and aspartic acid located in an n-th position from aspartic acid, the number of pairs (lys-lys) of lysine and lysine located in an n-th position from lysine, and the number of pairs (lys-asp) of lysine and aspartic acid located in an n-th position from lysine are counted.

For example, in this embodiment, the number of other amino acids located in the position at the distance n from aspartic acid and the number of other amino acids located in the position at the distance n from lysine are counted. Here, the other amino acids include leucine, aspartic acid, and lysine.

For example, in the cyclic peptide 20 illustrated in FIG. 2, there is only one pair 21 as the "leu-leu" pair in which n = 1. For example, in the cyclic peptide 20, with reference to certain leucine, the total number of leucines arranged in a first position from reference leucine is one.

Furthermore, in the cyclic peptide 20, there are three pairs 22, 23, and 24 as the "leu-lys" pair in which n = 1. For example, in the cyclic peptide 20, with reference to certain leucine, the total number of lysines arranged in a first position from reference leucine is three.

Similarly, in the cyclic peptide 20, there is one "leu-leu" pair in which n = 2. For example, in the cyclic peptide 20, with reference to certain leucine, the total number of leucines arranged in a second position from reference leucine is one.

Furthermore, in the cyclic peptide 20, there is one "leu-leu" pair in which n = 3. For example, in the cyclic peptide 20, with reference to certain leucine, the total number of leucines arranged in a third position from reference leucine is one.

In this manner, in this embodiment, possible combinations (pairs) of types of amino acids are specified in a plurality of amino acids included in the cyclic peptide 20. Then, in this embodiment, the matrix in which the types of the amino acids in the specified pair, the distance between the amino acids included in the pair, and the number of pairs for each distance are associated with one another is made the feature amount 30.

Therefore, according to this embodiment, it is possible to create the feature amount specialized for the cyclic peptide, and it is possible to reflect the feature of the structure of the cyclic peptide to the feature amount with high accuracy. Therefore, according to this embodiment, it is possible to contribute to acceleration of drug discovery by machine learning using this feature amount.

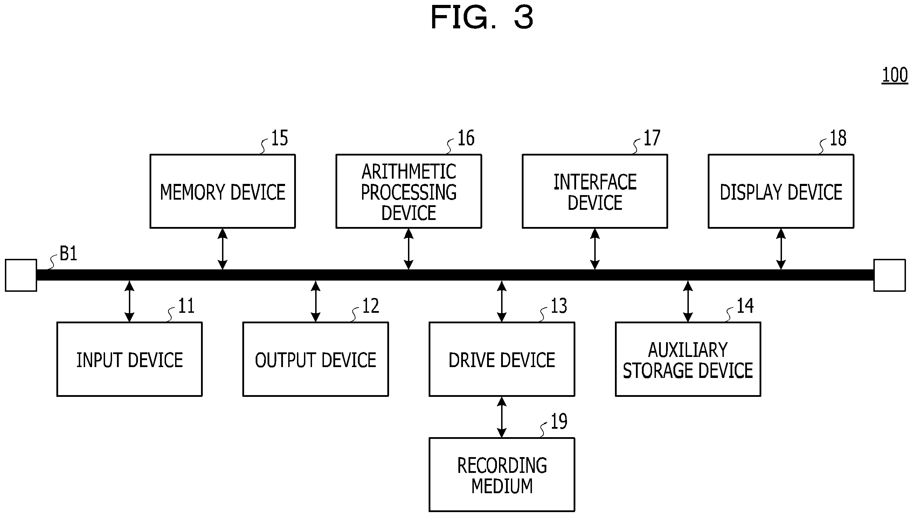

Hereinafter, a hardware configuration of the feature amount calculation device 100 of this embodiment is described with reference to FIG. 3. FIG. 3 is a diagram illustrating an example of the hardware configuration of the feature amount calculation device.

The feature amount calculation device 100 of this embodiment is a computer including an input device 11, an output device 12, a drive device 13, an auxiliary storage device 14, a memory device 15, an arithmetic processing device 16, and an interface device 17 mutually connected to one another via a bus B1.

The input device 11 is a device for inputting various types of information, and is implemented by, for example, a keyboard, a pointing device and the like. The output device 12 is for outputting various types of information, and is implemented by, for example, a display and the like. The interface device 17 includes a local area network (LAN) card and the like, and is used for connecting to a network.

The feature amount calculation program that implements the feature amount calculation unit 110 included in the feature amount calculation device 100 is at least part of various programs that control the feature amount calculation device 100. The feature amount calculation program is provided by, for example, distribution of a recording medium 18, download from the network and the like. As the recording medium 18 recording the feature amount calculation program, it is possible to use various types of recording media such as a recording medium that optically, electrically, or magnetically records information such as a compact disk read only memory (CD-ROM), a flexible disk, and a magneto-optical disc, a semiconductor memory that electrically records information such as a ROM and a flash memory and the like.

When the recording medium 18 that records the feature amount calculation program is set in the drive device 13, the feature amount calculation program recorded in the recording medium 18 is installed in the auxiliary storage device 14 from the recording medium 18 via the drive device 13. The feature amount calculation program downloaded from the network is installed in the auxiliary storage device 14 via the interface device 17.

The auxiliary storage device 14 stores the feature amount calculation program installed in the feature amount calculation device 100, and also stores various required files, data and the like by the feature amount calculation device 100. The memory device 15 reads the feature amount calculation program from the auxiliary storage device 14 at startup of the feature amount calculation device 100, and stores the same. Then, the arithmetic processing device 16 implements various types of processing to be described later in accordance with the feature amount calculation program stored in the memory device 15.

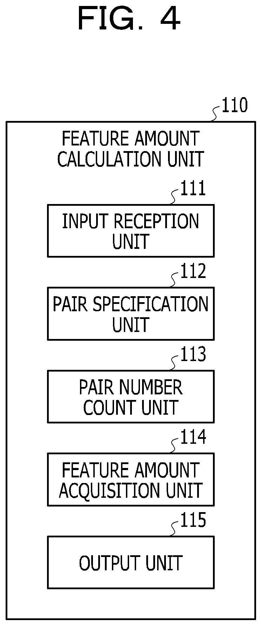

Next, a function of the feature amount calculation unit 110 of this embodiment is described with reference to FIG. 4. FIG. 4 is a diagram for illustrating the function of the feature amount calculation unit of the first embodiment.

The feature amount calculation unit 110 of this embodiment includes an input reception unit 111, a pair specification unit 112, a pair number count unit 113, a feature amount acquisition unit 114, and an output unit 115.

The input reception unit 111 receives various inputs to the feature amount calculation device 100. For example, the input reception unit 111 receives the structure specifying information 10 input to the feature amount calculation device 100.

The pair specification unit 112 specifies a pair in which amino acids are at a specified distance with reference to the structure specifying information 10.

The pair number count unit 113 counts the number of specified pairs included in the cyclic peptide.

The feature amount acquisition unit 114 acquires the feature amount in which the specified pair, the distance between the amino acids included in the pair, and the number counted by the pair number count unit 113 are represented as the matrix.

The output unit 115 outputs the feature amount acquired by the feature amount acquisition unit 114 to an external device such as the information processing device 200.

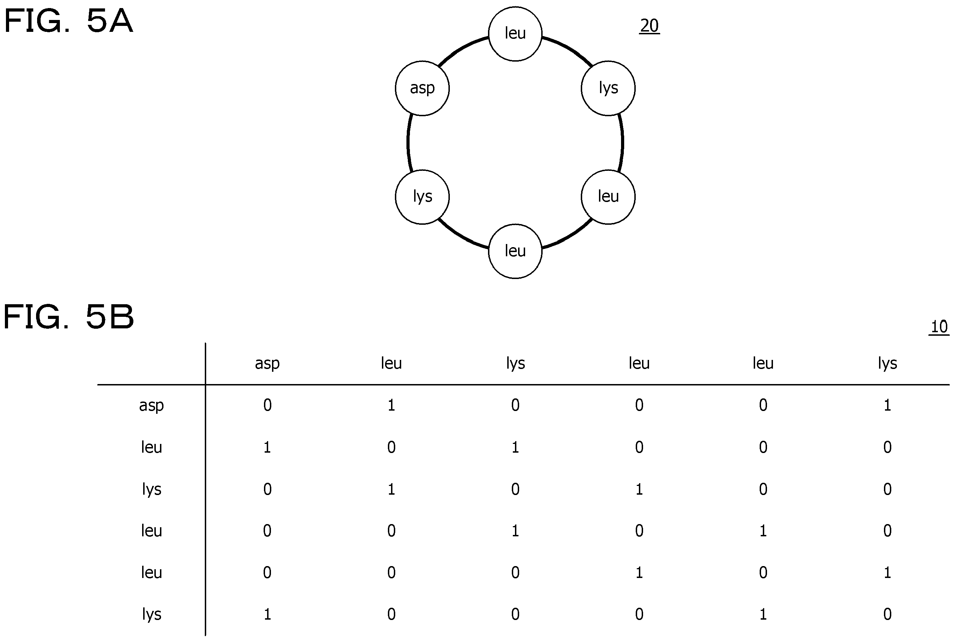

Next, the structure specifying information 10 of this embodiment is described with reference to FIG. 5. FIG. 5 (i.e., FIGs. 5A and 5B) is a diagram for illustrating the structure specifying information. FIG. 5A illustrates an example of the cyclic peptide, and FIG. 5B illustrates an example of the structure specifying information specifying the structure of the cyclic peptide.

The structure specifying information 10 of this embodiment is information including a type of the amino acid included in the cyclic peptide 20 and a type of an amino acid next to a certain amino acid.

For example, the cyclic peptide 20 includes three types of amino acids, which are aspartic acid, leucine, and lysine, as illustrated in FIG. 5A. Furthermore, the cyclic peptide 20 includes six amino acids.

The structure specifying information 10 is a matrix indicating the sequence of the amino acid included in the cyclic peptide 20, and components in each column and each row indicate whether the amino acids indicated by each column and row are next to each other.

In this embodiment, in the structure specifying information 10, in a case where a component in each column and each row is "0", this indicates that the amino acids indicated by each column and each row are not next to each other (distance n = 2 or longer), and in a case where a component in each column and each row is "1", this indicates that the amino acids indicated by each column and each row are next to each other (distance n = 1).

For example, in the structure specifying information 10 in FIG. 5B, a component in first column and second row and a component in first column and sixth row are "1", and it is understood that aspartic acid is next to leucine and lysine in the cyclic peptide 20. Furthermore, in the structure specifying information 10, a component in second column and second row and a component in second column and third row are "1", and it is understood that leucine arranged next to aspartic acid is also next to lysine in the cyclic peptide 20.

The structure specifying information 10 of this embodiment may be created in advance by, for example, a user of the information processing device 200 and the like and input to the feature amount calculation device 100.

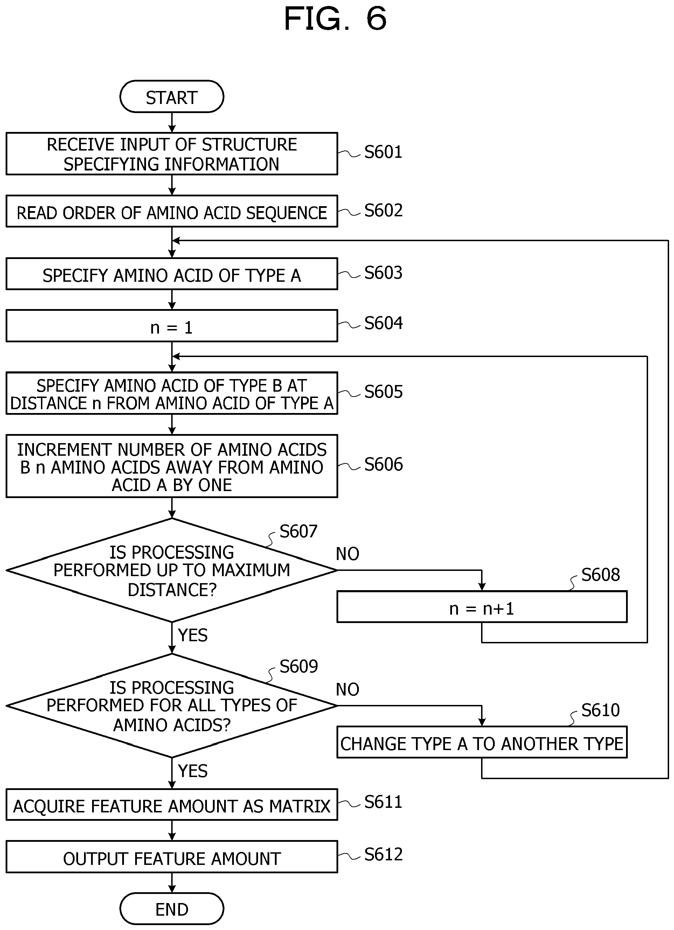

Next, processing of the feature amount calculation device 100 of this embodiment is described with reference to FIG. 6. FIG. 6 is a flowchart for illustrating the processing of the feature amount calculation device of the first embodiment.

The feature amount calculation unit 110 of the feature amount calculation device 100 of this embodiment receives an input of the structure specifying information 10 by the input reception unit 111 (step S601). Subsequently, the feature amount calculation device 100 reads order of the sequence of amino acids from the structure specifying information 10 by the pair specification unit 112 (step S602).

Subsequently, the pair specification unit 112 specifies a certain type of amino acid (first atomic group), which is one of the amino acids included in the pair, from the sequence of amino acids indicated by the structure specifying information 10 (step S603).

In the following description, a type of the amino acid specified at step S603 is sometimes represented by "A", and the amino acid of the type specified at step S603 is sometimes represented by an amino acid A.

Subsequently, the pair specification unit 112 sets a value of n indicating the distance between the amino acid A and an amino acid paired with the amino acid A to "1" (step S604).

Subsequently, the pair specification unit 112 specifies an amino acid (second atomic group) arranged n amino acids away from the amino acid A, from the sequence of amino acids indicated by the structure specifying information 10 (step S605).

In the following description, a type of the amino acid specified at step S605 is sometimes represented by "B", and the amino acid of the type specified at step S605 is sometimes represented by an amino acid B.

For example, in the sequence of amino acids indicated by the structure specifying information 10, the pair specification unit 112 specifies the amino acid of the type "B" arranged at a distance n from the amino acid of the type "A" with reference to the amino acid of the type "A".

Subsequently, the feature amount calculation unit 110 counts the number of amino acids B n amino acids away from the amino acid A by the pair number count unit 113 (step S606).

For example, the pair number count unit 113 counts the number of pairs including the amino acid A and the amino acid B located in an n-th position from the amino acid A.

Subsequently, the feature amount calculation unit 110 determines whether the processing from step S603 to step S606 is performed until the value of n reaches a maximum value in the sequence of amino acids indicated by the structure specifying information 10 (step S607). The maximum value of n may be the number of amino acids included in the cyclic peptide indicated by the structure specifying information 10.

At step S607, in a case where the value of n is not maximized, the feature amount calculation unit 110 sets n = n + 1 (step S608) and returns to step S605.

At step S607, in a case where the value of n is maximized, the feature amount calculation unit 110 determines whether the processing from step S604 to step S608 is performed for all the types of amino acids included in the structure specifying information 10 (step S609).

At step S609, in a case where the processing is not performed for all the types of amino acids, the feature amount calculation unit 110 sets a type different from the type specified at step S603 to type "A" (step S610), and returns to step S604.

At step S609, in a case where the processing is performed for all the types of amino acids, the feature amount calculation unit 110 acquires the feature amount 30 in which the number acquired by the pair number count unit 113 is represented by a matrix by the feature amount acquisition unit 114 (step S611).

Subsequently, the feature amount calculation unit 110 outputs the acquired feature amount 30 to an external device such as the information processing device 200 by the output unit 115 (step S612), and finishes the processing.

In this manner, the feature amount calculation device 100 of this embodiment executes processing of receiving the structure specifying information of specifying each type of a plurality of atomic groups (amino acids) and the sequence of the plurality of atomic groups regarding the cyclic peptide, which is a cyclic molecule in which the plurality of atomic groups classified into a plurality of types is cyclically sequenced. Furthermore, when the feature amount calculation device 100 receives the structure specifying information, this executes processing of specifying an optional first type (amino acid A) and an optional second type (amino acid B) out of the plurality of types, and processing of specifying one or a plurality of first atomic groups classified into the first type and one or a plurality of second atomic groups classified into the second type out of the plurality of atomic groups, on the basis of the structure specifying information. Moreover, the feature amount calculation device 100 executes processing of calculating the number of pairs of the first atomic group and the second atomic group in which a distance n therebetween in the sequence of the first atomic group and the second atomic group is a predetermined distance, on the basis of the structure specifying information.

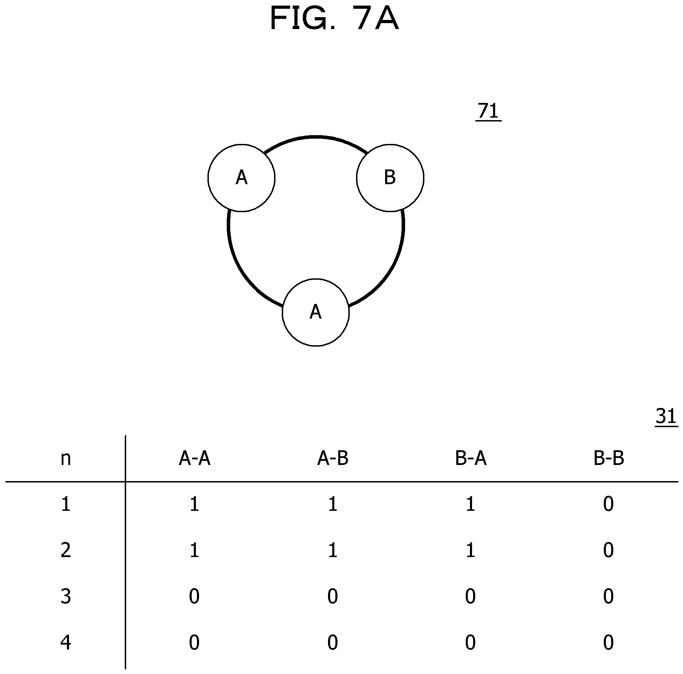

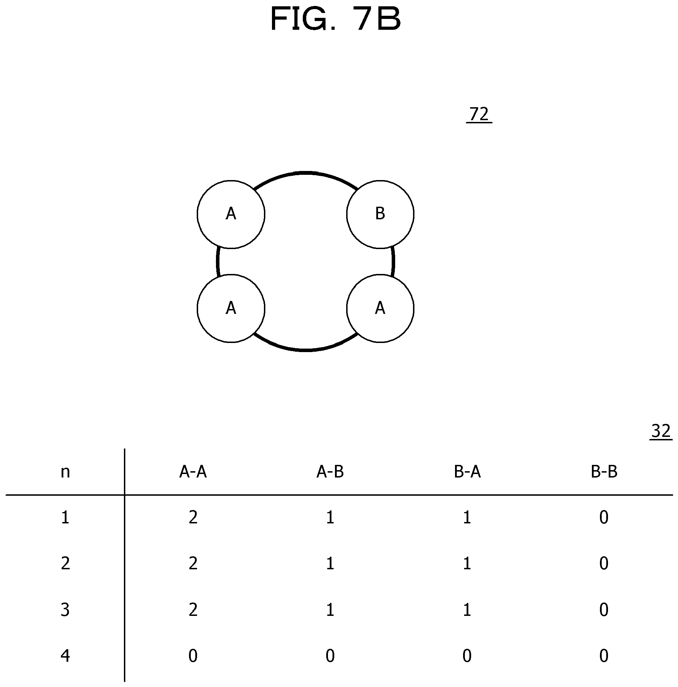

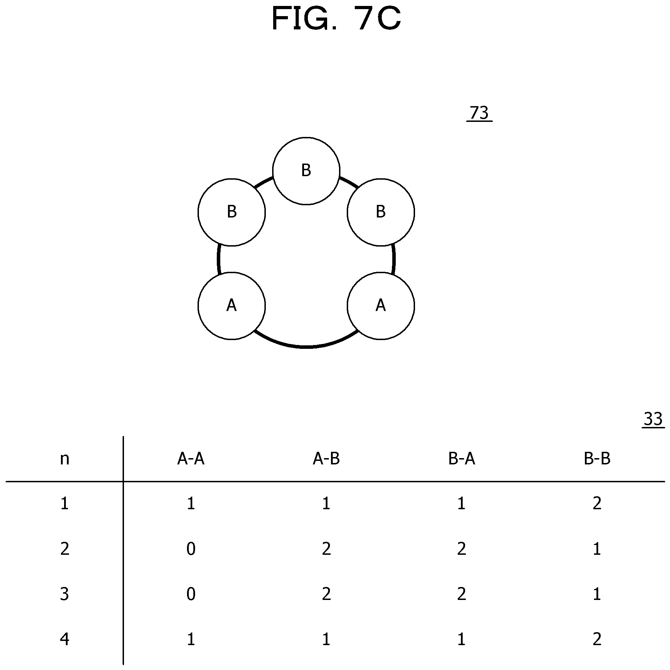

It is possible to calculate mutual similarity of a plurality of cyclic peptides on the basis of the feature amount acquired by applying this embodiment, and apply the feature amount to processing of machine learning and the like. FIG. 7A is a first diagram for illustrating processing using the feature amount. FIG. 7B is a second diagram for illustrating processing using the feature amount. FIG. 7C is a third diagram for illustrating processing using the feature amount.

FIG. 7A, FIG. 7B, and FIG. 7C illustrate a case where the feature amount is acquired by applying this embodiment regarding a cyclic peptide 71, a cyclic peptide 72, and a cyclic peptide 73, respectively.

A feature amount 31 illustrated in FIG. 7A is a feature amount acquired by applying this embodiment regarding the cyclic peptide 71 including two amino acids A and one amino acid B. Furthermore, a feature amount 32 illustrated in FIG. 7B is a feature amount acquired by applying this embodiment regarding the cyclic peptide 72 including three amino acids A and one amino acid B. Furthermore, a feature amount 33 illustrated in FIG. 7C is a feature amount acquired by applying this embodiment to the cyclic peptide 73 including two amino acids A and three amino acids B.

In this embodiment, the information processing device 200 calculated the similarity of the cyclic peptides 71, 72, and 73 on the basis of the feature amounts 31, 32, and 33 calculated by the feature amount calculation device 100. For example, in this embodiment, the similarity of the cyclic peptides 71, 72, and 73 was calculated using a cosine similarity formula. The cosine similarity formula is a method of regarding a matrix as a vector in one row and calculating the similarity from an angle formed between the vectors.

In the example of FIGs. 7A to 7C, the similarity between the cyclic peptide 71 and the cyclic peptide 72 was 0.77, the similarity between the cyclic peptide 71 and the cyclic peptide 73 was 0.51, and the similarity between the cyclic peptide 72 and the cyclic peptide 73 was 0.50.

In this manner, by using the feature amount to which this embodiment is applied, the similarity between the cyclic peptides may be compared and examined regardless of the size and the like of the cyclic peptide. Furthermore, the information processing device 200 may perform machine learning on the basis of teacher data including attribute values of known cyclic peptides, and estimate the attribute values of the cyclic peptides 71, 72, and 73 on the basis of the feature amounts 31, 32, and 33 calculated by the feature amount calculation device 100. Furthermore, the information processing device 200 may perform machine learning on the basis of information regarding the feature amounts 31, 32, and 33 and the attribute values of the cyclic peptides 71, 72, and 73. Note that, the information processing device 200 is a computer including an input device, an output device, a drive device, an auxiliary storage device, a memory device, an arithmetic processing device, and an interface device mutually connected to one another via a bus.

### (Second Embodiment)

Hereinafter, a second embodiment is described with reference to the drawings. The second embodiment is different from the first embodiment in specifying whether a distance n between amino acids is made a distance in a first direction of a cycle in a cyclic molecule or a distance in a second direction opposite to the first direction. In the description of the second embodiment below, the difference from the first embodiment is described, and a component having a functional configuration similar to that in the first embodiment is denoted by a reference sign similar to the reference sign used in the description of the first embodiment, and the description thereof is omitted.

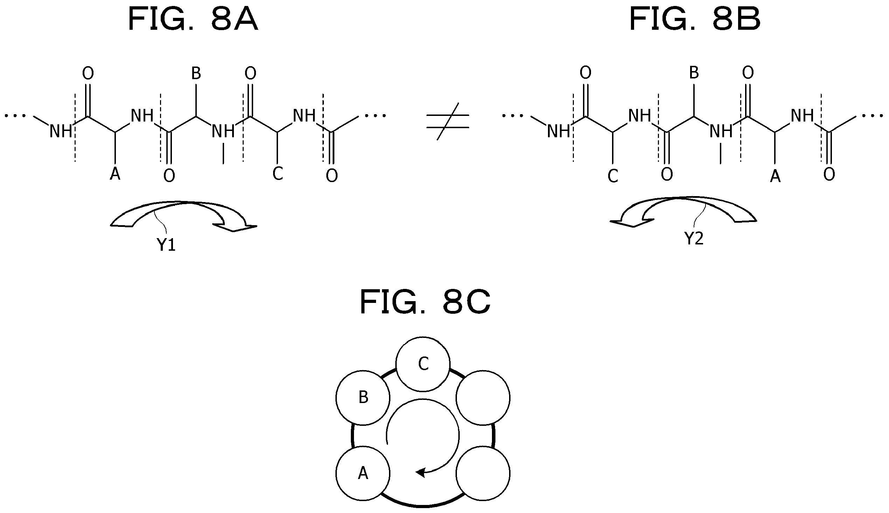

FIGs. 8A to 8C are diagrams for illustrating a feature amount of the second embodiment. FIGs. 8A and 8B illustrate a state in which, in a sequence of amino acids, an amino acid of a type "A", an amino acid of a type "B", and an amino acid of a type "C" are bonded by amide bond (-NHCO-).

In this case, since the amino acids are bonded to each other by the amide bond, a structure is different between a case where the amino acid A, the amino acid B, and the amino acid C are sequenced in this order in a clockwise direction on the drawing and a case where the amino acid A, the amino acid B, and the amino acid C are sequenced in this order in a counterclockwise direction on the drawing.

FIG. 8A illustrates an example of a case where the amino acid A, the amino acid B, and the amino acid C are sequenced in this order in the clockwise direction (direction of arrow Y1). In this case, an N-terminus of the amino acid A is bonded to a C-terminus of the amino acid B, and an N-terminus of the amino acid B is bonded to a C-terminus of the amino acid C.

FIG. 8B illustrates a case where the amino acid A, the amino acid B, and the amino acid C are sequenced in this order in the counterclockwise direction (direction of arrow Y2). In this case, a C-terminus of the amino acid A is bonded to an N-terminus of the amino acid B, and a C-terminus of the amino acid B is bonded to an N-terminus of the amino acid C.

Therefore, a pair of the amino acid A and the amino acid B with a distance n = 1 in FIG. 8A and a pair of the amino acid A and the amino acid B with a distance n = 1 in FIG. 8B have different structures.

In this embodiment, focusing on this point, when determining the pair of amino acids, it is specified whether the distance between the amino acids is a distance in the clockwise direction or a distance in the counterclockwise direction. For example, in this embodiment, together with the structure specifying information 10, an input of direction specifying information specifying whether the distance between the amino acids is the distance in the clockwise direction or the distance in the counterclockwise direction is accepted.

Then, in this embodiment, a feature amount of a cyclic peptide is calculated on the basis of the structure specifying information 10 and the direction specifying information.

Furthermore, in this embodiment, since the direction of the distance between the amino acids is specified by the direction specifying information, even if the amino acids included in the pairs are the same, they are counted as different pairs.

A cyclic peptide 80 illustrated in FIG. 8C includes the amino acid A, the amino acid B, the amino acid C, and two other amino acids.

In this case, in a case where the distance between the amino acids is made the distance in the clockwise direction, a pair of the amino acid A and the amino acid C is a pair of the amino acid A and the amino acid C two amino acids away from the amino acid A in the clockwise direction and a pair of the amino acid C and the amino acid A three amino acids away from the amino acid C in the clockwise direction.

For example, in the cyclic peptide 80, in a case where the distance in the clockwise direction is made the distance between the amino acids, the pair including the amino acid A and the amino acid C is the pair of the amino acid A and the amino acid C with a distance n = 2 and the pair of the amino acid C and the amino acid A with a distance n = 3.

In this manner, in this embodiment, even when the types of the amino acids included in the pairs are the same, the direction when specifying the distance is specified, so that these pairs are counted separately. Therefore, in this embodiment, the sequence of the amino acids may be expressed more accurately.

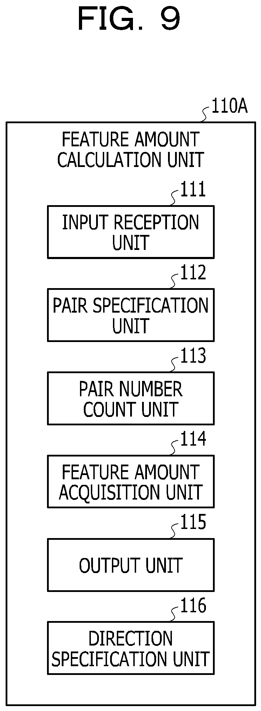

Hereinafter, a functional configuration of a feature amount calculation unit 110A of this embodiment is described with reference to FIG. 9. FIG. 9 is a diagram for illustrating a function of the feature amount calculation unit of the second embodiment.

The feature amount calculation unit 110A of this embodiment includes an input reception unit 111, a pair specification unit 112A, a pair number count unit 113, a feature amount acquisition unit 114, an output unit 115, and a direction specification unit 116.

The pair specification unit 112A specifies another amino acid located in a position at a distance n from a certain amino acid in a direction specified by the direction specification unit 116 as an amino acid paired with the certain amino acid.

The direction specification unit 116 specifies the direction when counting the distance between the amino acids in the cyclic peptide on the basis of the direction specifying information input from the information processing device 200 and the like.

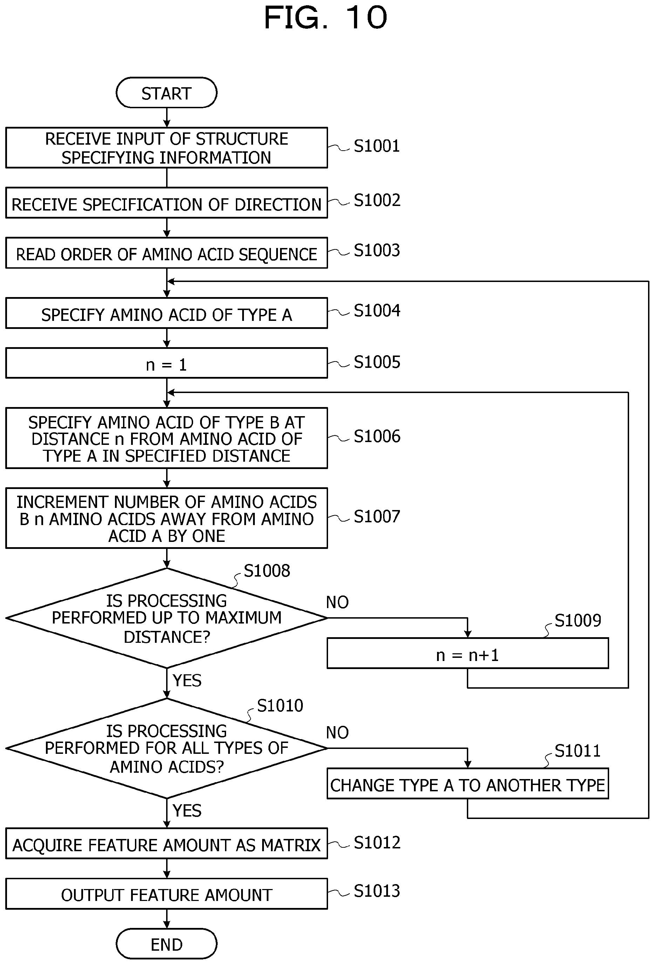

Hereinafter, processing of the feature amount calculation unit 110A of this embodiment is described with reference to FIG. 10. FIG. 10 is a flowchart for illustrating processing of the feature amount calculation device of the second embodiment.

The feature amount calculation unit 110A of this embodiment receives an input of the structure specifying information 10 by the input reception unit 111 (step S1001). Subsequently, the feature amount calculation unit 110A receives an input of the direction specifying information by the input reception unit 111 (step S1002).

Since processing from step S1003 to step S1005 in FIG. 10 is similar to the processing from step S602 to step S604 in FIG. 6, the description thereof is omitted.

Following step S1005, the feature amount calculation unit 110A refers to the direction specifying information input at step S1002 by the pair specification unit 112A, specifies the amino acid arranged in a position at the distance n in the specified direction from the amino acid of the type "A" (step S1006), and shifts to step S1007.

Since the processing from step S1007 to step S1013 in FIG. 10 is similar to the processing from step S606 to step S612 in FIG. 6, the description thereof is omitted.

In this manner, in this embodiment, when specifying another amino acid located in the position at the distance n from a certain amino acid, the another amino acid at the distance n is specified in the specified direction. Therefore, according to this embodiment, the structure of the cyclic peptide formed by the sequence of the amino acids may be reflected in the feature amount with high accuracy.

## Claims

1. A feature amount calculation program comprising instructions which, when executed by a computer, cause the computer to execute processing comprising:
receiving structure specifying information that specifies a type of each of a plurality of atomic groups and a sequence of the plurality of atomic groups regarding a cyclic molecule in which the plurality of atomic groups classified into a plurality of types is cyclically sequenced;
specifying an optional first type and an optional second type in the plurality of types;
specifying, on the basis of the structure specifying information, one or a plurality of first atomic groups classified into the first type and one or a plurality of second atomic groups classified into the second type out of the plurality of atomic groups; and
calculating, on the basis of the structure specifying information, a number of pairs of the first atomic group and the second atomic group in which a mutual distance in the sequence between the first atomic group and the second atomic group is a predetermined distance.

2. The non-transitory computer-readable recording medium according to claim 1, further causing the computer to execute the process comprising:
receiving direction specifying information that specifies either a first direction along a cycle of the cyclic molecule or a second direction along the cycle opposite to the first direction; and
calculating the distance in the direction specified by the direction specifying information.

3. The non-transitory computer-readable recording medium according to claim 1 or 2, wherein each of the plurality of atomic groups is an amino acid, and the cyclic molecule is a cyclic peptide.

4. A feature amount calculation method implemented by a computer, the feature amount calculation method comprising:
receiving structure specifying information that specifies a type of each of a plurality of atomic groups and a sequence of the plurality of atomic groups regarding a cyclic molecule in which the plurality of atomic groups classified into a plurality of types is cyclically sequenced;
specifying an optional first type and an optional second type in the plurality of types;
specifying, on the basis of the structure specifying information, one or a plurality of first atomic groups classified into the first type and one or a plurality of second atomic groups classified into the second type out of the plurality of atomic groups; and
calculating, on the basis of the structure specifying information, a number of pairs of the first atomic group and the second atomic group in which a mutual distance in the sequence between the first atomic group and the second atomic group is a predetermined distance.

5. A feature amount calculation apparatus comprising:
a reception unit of receiving structure specifying information that specifies a type of each of a plurality of atomic groups and a sequence of the plurality of atomic groups regarding a cyclic molecule in which the plurality of atomic groups classified into a plurality of types is cyclically sequenced;
a first processing unit of specifying an optional first type and an optional second type in the plurality of types;
a second processing unit of specifying, on the basis of the structure specifying information, one or a plurality of first atomic groups classified into the first type and one or a plurality of second atomic groups classified into the second type out of the plurality of atomic groups; and
a third processing unit of calculating, on the basis of the structure specifying information, a number of pairs of the first atomic group and the second atomic group in which a mutual distance in the sequence between the first atomic group and the second atomic group is a predetermined distance.
